# EUROPEAN PATENT SPECIFICATION

(11) **EP 3 814 320 B1**
(45) Date of publication and mention of the grant of the patent: **06.12.2023**
(21) Application number: 19753195.7
(22) Date of filing: 28.06.2019
(51) Int. Cl.: C07C 273/12

(54) **CONVERSION PROCESS OF ANHYDROUS OFF-GAS STREAM COMING FROM MELAMINE SYNTHESIS PLANTS INTO UREA**
UMWANDLUNGSVERFAHREN VON WASSERFREIEM ABGASSTROM AUS MELAMINSYNTHESEANLAGEN IN HARNSTOFF
PROCÉDÉ DE CONVERSION D'UN FLUX D'EFFLUENT GAZEUX ANHYDRE PROVENANT D'INSTALLATIONS DE SYNTHÈSE DE MÉLAMINE EN URÉE

(30) Priority: 29.06.2018 IT 201800006795
(43) Date of publication of application: 05.05.2021
(73) Proprietor: Proman AG, 8832 Wollerau Schwyz (CH)
(72) Inventor: CAVUOTI, Giacomo, 47923 Rimini (IT); DE AMICIS, Alberto, 20097 San Donato Milanese (MI) (IT); DI RUOCCO, Giuseppe, 23900 Lecco (IT)
(74) Representative: De Gregori, Antonella
(86) International application number: PCT/IB2019/055506
(87) International publication number: WO 2020/003234

(56) References cited:
- WO-A1-98/08808
- US-B1- 6 586 629

## Description

The present invention relates to a process for the conversion of anhydrous off-gases into urea, said off-gases coming from melamine synthesis plants.

The present invention therefore also relates to a direct production process of urea, starting from anhydrous off-gases coming from melamine synthesis plants and the recycling of urea to the melamine plant, in addition to the relative plant.

As is known, melamine is produced by low-pressure (less than 10 bars) and high-temperature (350-450°C) trimerization with catalysts or by high-pressure (50.6625-121.59 bar (50-120atm)) and high-temperature (350-450°C) trimerization of urea, without catalysts:

6 CO(NH₂)₂ → C₃H₆N₆ + 3CO₂ + 6NH₃

The reaction is carried out in excess of ammonia.

The by-product of the reaction, consisting of an anhydrous gaseous stream of ammonia and carbon dioxide in a molar ratio greater than 2 (the off-gases) is generally recycled to a urea plant which converts said by-product back into urea.

This high-pressure and high-temperature gaseous off-gas stream also contains small percentages of melamine in gas phase which tends to desublimate at a temperature below 354°C.

In the processes according to the state of the art, the gaseous off-gas stream is cooled and purified by the melamine entrained by the injection of water, urea or ammonia according to the provisions of the specific process considered. The cooled and cleaned off-gases are then recycled to the urea plant.

The transfer of these off-gases to a urea plant separate from the melamine plant creates considerable problems of coupling and constraint between the running regimes of the two plants, above all in the starting, stopping phases, operating at reduced regime or in the case of anomalies. It is often necessary, for example, to provide storage volumes or off-gas disposal methods if the melamine plant is running while the urea plant is stationary. Furthermore, if the off-gas washing is carried out with water, the off-gases become saturated with water vapour which, as this is a reaction product of the formation of urea

2NH₃ + CO₂ → CO(NH₂)₂ + H₂O

drastically reduces the conversion of the reagents to urea.

The problem is even more relevant when the off-gases of the melamine plant are at a lower pressure than the pressure present in the urea synthesis reactor (as happens in almost all existing processes).

This problem is completely analogous to that which arises in the recycling of unreacted ammonia and CO₂, separated from the medium- (20 bars) and low-pressure (1-5 bars) sections of the urea plant, to the high-pressure (150 - 210 bars) synthesis reactor in Urea plants, as widely discussed in literature and as demonstrated by the design and operation of Urea plants. The great difficulty in recycling unreacted ammonia and CO₂ is basically linked to the possibility of the formation of solid ammonium carbamate which takes place at temperatures lower than the crystallization temperature of anhydrous ammonium carbamate, i.e. at temperatures lower than 152 C.

This problem is particularly serious when there are "cold points" in the system. These "cold points" can always be present in equipment and transport lines, even if appropriately insulated and heated.

In view of the above-mentioned technological difficulties present in the recycling of unreacted ammonia and CO₂, various alternative solutions have been proposed in the state of the art, for the recycling of unreacted ammonia and CO₂ at medium and low pressure. In particular:
a. "One-through" processes without recycling: the carbamate that is formed by the crystallization of CO₂ and ammonia is not recycled to the synthesis of urea and is used for the production of other fertilizers (e.g. ammonium nitrate).
   This is a disadvantageous method as most of the reagents are discharged as by-products for the production of fertilizers with lower added value.
b. Recycling with auxiliary fluid: the carbamate recycling is carried out in the liquid phase, after condensation and pumping with the aid of an auxiliary fluid (for example mineral oil), which allows an internal temperature control of the streams and recycling pumps, preventing the formation of cold points and the consequent formation of solid carbamate deposits. This solution has obvious disadvantages linked to the use and management of the auxiliary fluid with possible pollution of the final product.
c. "Hot gas recycle": high-temperature recycling in gas phase. The unreacted ammonia and CO₂ are recycled to the reactor, keeping them in gas phase at high temperature (well above 152°C) through compressors made of materials suitable for high temperatures and corrosive atmospheres. This method involves serious burdens both in terms of installation costs for the addition of expensive turbochargers and also in terms of energy costs due to the need for compressing a gas instead of pumping a liquid.
d. Recycling after ammonia/CO₂ separation: Ammonia and CO₂ are recycled at the synthesis pressure, after separating these two compounds and possibly also separating the water, so as to avoid technological problems relating to the compression of the mixture which, as already indicated, can give rise to the deposit of solid salts. In this case an actual separation system is used, often proposed with the use of auxiliary substances (organic amines), which involves considerable energy costs due to the decomposition of the ammonium salts fed (about 500 kcal / kg).
e. Recycling in aqueous solution: unreacted ammonia and CO₂ are pumped to the high-pressure reactor in aqueous solution in the form of ammonium carbamate and carbonates.

This solution involves a considerable reduction in the conversion of the urea reactor due to the water (reaction product) which seriously hinders the reaction (passing from a possible 85% conversion under anhydrous conditions, to conversion values of 63-65%), but the drastic lowering of the crystallization temperatures and vapour pressure of the recycling streams is obtained. This is the method currently adopted in almost all existing Urea plants.

The present invention is therefore particularly advantageous in running contexts of the two separate plants, melamine and urea, mentioned above and above all applies to contexts in which the off-gases of the melamine plant are at a lower pressure than the pressure present in the reactor for the synthesis of urea.

This is now true for most modern melamine synthesis processes which, for obvious reasons of reducing plant and operating costs, tend to be increasingly low-pressure processes. WO98/08808 discloses a process for the preparation of urea whereby an off-gas stream released during the synthesis of melamine which consists predominantly of ammonia and carbon dioxide is introduced into at least one high-pressure section of a urea stripping plant and is used in the synthesis of urea

The off-gases resulting from the synthesis of melamine are available within a pressure range that varies from atmospheric pressure to 120 bars, preferably lower than 100 bars and even more preferably within the range of 70-90 bars, therefore well below the synthesis pressure of the Urea reactors (150-210 bars).

Other gas compression systems among the pressures in question (from 70-90 bars to 150-210 bars) such as the use of ejectors are unrealistic from the point of view of energy consumption and the imbalance of the overall mass balances of the system due to the high flow-rates of the motor fluid required (as for example in US 6,586,629).

The objective of the present invention is therefore to overcome the above-mentioned drawbacks which characterize the state of the art.

A process has in fact been surprisingly found, for the direct conversion of the anhydrous off-gases, having a pressure lower than 120 bars, from melamine to urea plants, which is particularly efficient and which takes place, preferably, within the melamine plant, allowing the recycling of the urea thus produced to the melamine synthesis reactor.

The object of the present invention therefore relates to a process for the direct conversion into urea of an anhydrous off-gas stream coming from melamine synthesis plants, at a pressure lower than 120 bars, comprising the following steps:
a) a first isobar cooling step of the off-gas stream;
b) a transformation step of the cooled off-gas stream coming from step a) into ammonium carbamate and NH₃;
c) an isothermal pressurization step of the ammonium carbamate obtained in the previous step;
d) an isobar heating step of the product coming from step c);
e) a conversion step of the ammonium carbamate coming from step d) into urea.

The process for the direct conversion of anhydrous off-gases from melamine plants into urea according to the present invention is particularly advantageous in that it allows a high-efficiency recycling of the melamine synthesis by-products (off-gases) in the form of urea with extremely high conversions and it allows the melamine plant and urea plant to be substantially decoupled.

### a) Isobar cooling

In the first step of the process according to the present invention, the anhydrous off-gas stream subjected to isobar cooling comes from the melamine plant and more specifically from the melamine synthesis reactor or from the post-reactor optionally present in the melamine plant, or from both.

The anhydrous off-gas stream, having a pressure lower than 120 bars, preferably lower than 100 bars and even more preferably within the range 60-100 bars, is then isobarically cooled and optionally purified from the melamine residues contained therein by one of the methods known in the state of the art, for example through already existing waterless washing processes (with urea, or sublimation by cooling with ammonia, as described in IT1338957).

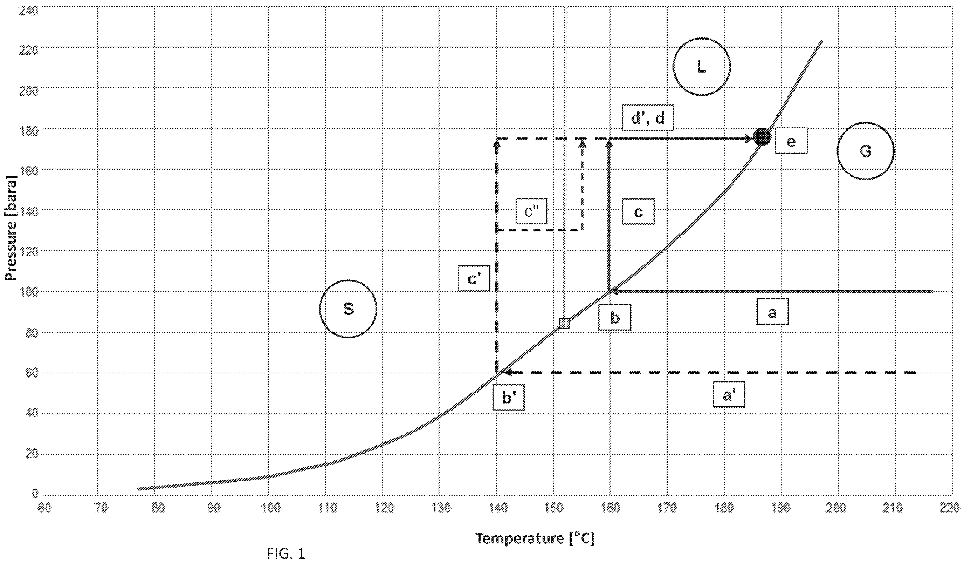

In particular, as described in detail hereunder, the process according to the present invention provides the above-mentioned unitary operations/steps a) -e) represented in figures 1, 2 and 3.

### b) Transformation step of the cooled off-gas stream coming from step a) into ammonium carbamate and NH₃.

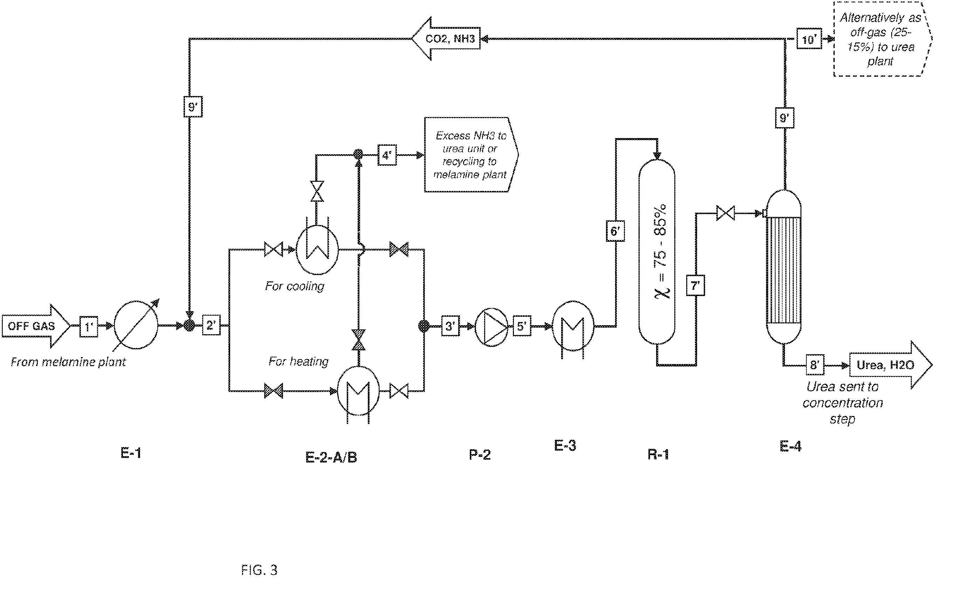

More specifically, step b) of the process according to the present invention is a condensation or desublimation step of the off-gases to ammonium carbamate. Depending, in fact, on whether the operating pressure of the melamine synthesis process is higher or lower than the triple point pressure of the ammonium carbamate:
- the off-gases are condensed in liquid phase in the form of a liquid solution of ammonium carbamate and NH₃, in a surface condenser;
- or CO₂ and NH₃ desublimate in solid phase in the form of solid ammonium carbamate in at least a pair of desublimators (shown in Figure 3 with E-2-AB) - for example of the switch-condenser type for phthalic anhydride - which operate alternatively during desublimation (Cooling-Mode) and pressurization/melting, (Heating-Mode) :

   (2+x) NH₃ (g) + CO₂ (g) → NH₂CONH₄ (l,s) + x NH₃ (g)

In both cases the excess ammonia is recovered in the gas phase.

### c) Isothermal pressurization

More specifically, in step c) of the process according to the present invention, the anhydrous ammonium carbamate, liquid or solid, obtained in the previous step b), is pressurized at a pressure suitable for the synthesis of urea (145-220 bars).

The pressurization is carried out with a continuous process through a pump (P-1) if the anhydrous ammonium carbamate is liquid, or with a discontinuous process by pressurization with CO₂ (and possibly NH₃) in the desublimator, if the anhydrous ammonium carbamate is obtained in solid form.

In this case, during heating (Heating Mode), the pressurization and heating can be carried out partially (see path c" in figure 1) or completely (see path c, figure 1) inside the desublimator.

As specified hereunder, the pressurization inside the desublimator can be advantageously carried out by means of the "liquid bottle" technique using compressed CO₂ (and possibly NH₃).

In this way the carbamate recycling pump is eliminated (path c), or its prevalence is greatly reduced (path c").

### d) Isobar heating

In step d) of the process according to the present invention, the anhydrous ammonium carbamate pressurized at a pressure (145-220 bars) suitable for the synthesis of urea in step c), is heated to a temperature suitable for a rapid synthesis of the urea (T = 180-200°C).

If the carbamate was obtained in solid phase, the heating process crosses the solid/liquid equilibrium curve with melting of the carbamate.

### e) Conversion of ammonium carbamate to urea

The anhydrous ammonium carbamate in liquid phase and under pressure and temperature conditions suitable for the efficient formation of urea (T = 180-200 °C, P = 145-220 bars), is introduced into a plug-flow reactor (R-1), preferably and advantageously fed from above (down-flow) with a residence time in the order of some tens of minutes in order to reach equilibrium. If the ammonium carbamate is fed to the reactor from the bottom (i.e. up-flow), traditional anti-recycling plates must be provided in the reactor.

The ammonium carbamate is thus converted very efficiently into urea with expected conversions of 75-85%, thanks to the anhydrous conditions and in relation to the operating temperature:

NH₂CONH₄ (l) → CO(NH₂)₂ (l) + H₂O (l)

The process for the direct conversion of anhydrous off-gases from melamine plants into urea according to the present invention is therefore particularly advantageous, as it allows, as indicated above, a high-efficiency recycling of melamine synthesis by-products to be obtained, i.e. anhydrous off-gases , having a pressure lower than 120 bars, preferably lower than 100 bars and even more preferably within the range 60-100 bars, producing urea with high conversions (75-85%), thanks to the absence of water in the reagents.

A further fundamental advantage is that this process according to the present invention allows the melamine plant to be substantially completely decoupled from the urea plant. There may be the need for recycling a small stream of ammonia if it cannot be recycled directly to the melamine plant.

Furthermore, as the urea synthesis reactor is fed with liquid carbamate (and not gaseous CO₂), it can be advantageously fed from above, improving the plug-flow characteristics of the internal fluid dynamics, without the need for the anti-recycling plates of standard reactors. The plates are introduced into the urea reactors with the main function of preventing the reflux of the high-density products (urea and water solutions: 1,000 - 1,200 kg/m3) compared to the low-density reagents (ammonia - about 600 kg/m³ , and CO₂ - about 200 kg/m³).

As previously indicated, moreover, the process according to the present invention has the further advantage of not necessarily requiring the purification of the off-gases from the sublimated melamine, as the urea produced by the process according to the present invention, directly in the plant melamine, is specifically intended to be recycled to the melamine synthesis reactor.

Finally, a further advantage of the process according to the present invention is that it allows the production of urea by means of an absolutely innovative way of recycling/recompression of the reactants (NH₃ and CO₂), not in the form of an aqueous solution as in all existing processes, but anhydrously, specifically in the case considered above starting from anhydrous off-gases, with a large increase in conversion (from 60-65% to 75-85%) and a consequent reduction in the total recycling (from -43% to -70%) and proportional energy savings of heat and electricity.

The conversions obtained with the process according to the present invention are in fact particularly advantageous if compared with the 60-65% conversions obtained in the current non-anhydrous urea synthesis reactors (molar ratio H₂O/CO₂ in the reagents is normally equal to 0.45-0.65).

Furthermore, the process and apparatus according to the present invention do not require the use of any motor fluid.

A further object of the present invention also relates to a plant for carrying out said process which comprises:
- a cooling device suitable for cooling an anhydrous off-gas stream, having a pressure lower than 120 bars, preferably lower than 100 bars and even more preferably within the range of 70-100 bars, coming from the melamine synthesis plant;
- a condenser or at least a pair of desublimators, respectively suitable for condensing or desublimating and pressurizing an off-gas stream coming from the cooling device into ammonium carbamate and ammonia;

- a pump suitable for pressurizing the ammonium carbamate coming from the condenser at a pressure ranging from 145 to 220 bars and sending said ammonium carbamate to a heating element or a pump suitable for sending the ammonium carbamate coming from the desublimator to said heating element;
- a heating element suitable for heating the pressurized ammonium carbamate to a reaction temperature T ranging from 180 to 200°C;
- a reactor suitable for converting the ammonium carbamate into urea.

If the pair of desublimators is present, the plant according to the present invention provides that the pair of desublimators send the ammonium carbamate directly to the heater element, in the absence of a pump.

In order to better understand the features of the present invention, the following description refers to the following figures:
- Figure 1: a schematic representation of the steps of the process in relation to temperature and pressure based on a P-T state diagram of pure ammonium carbamate;
- Figure 2: simplified diagram of a first embodiment of the process according to the present invention;
- Figure 3: simplified diagram of a second embodiment of the process according to the present invention.

In accordance with Figure 1, the variations in pressure and temperature are represented, of the steps of the process according to the present invention.

More specifically, the continuous line shown in Figure 1 is representative of steps a) -c) and partially d) of the process according to the present invention, when the operating pressure of the melamine synthesis process is higher than the triple point pressure (in Figure 1 indicated with ■) of ammonium carbamate.

The dotted line is representative of steps a) -c), and partially d) of the process according to the present invention, identified in Figure 1 with the letters a') - c') and partially d', when the operating pressure of the melamine synthesis process is lower than the triple point pressure (■) of ammonium carbamate.

In particular, during heating (Heating Mode), the pressurization and heating can be carried out partially (path c" in Figure 1) or completely (path c' in Figure 1) inside the desublimator. If the pressurization and heating are carried out only partially inside the desublimator (first section c' and first section c" respectively), the remaining part of pressurization and heating is carried out externally by means of a pump and a standard exchanger (second section c" and d').

The pressurization inside the desublimator can be advantageously carried out with the "liquid bottle" technique using compressed CO₂ (and possibly NH₃) and in this way the carbamate recycling pump (path c') can be eliminated, or its prevalence (path c") is greatly reduced.

Steps d) and e), represented by a continuous line, are common to both processes.

The point (•) in Figure 1 represents the urea synthesis pressure and temperature conditions.

In short, the solid line represents the solution:
off-gas pressure > triple point carbamate pressure (■): path a-b-c-d
a) off-gas isobar cooling
b) off-gas condensation to liquid ammonium carbamate and liquid ammonia
c) carbamate + ammonia compression with pump
d) heating of liquid carbamate/ammonia up to the Urea synthesis conditions.

The dotted line represents the solution:

off-gas pressure < triple point carbamate pressure (■): path a'-b'-c'-d'
a) off-gas isobar cooling
b) off-gas desublimation/condensation to solid ammonium carbamate and liquid ammonia
c) carbamate + ammonia isothermal compression in confined volume
d) heating of carbamate/ammonia with carbamate melting up to the Urea synthesis conditions.

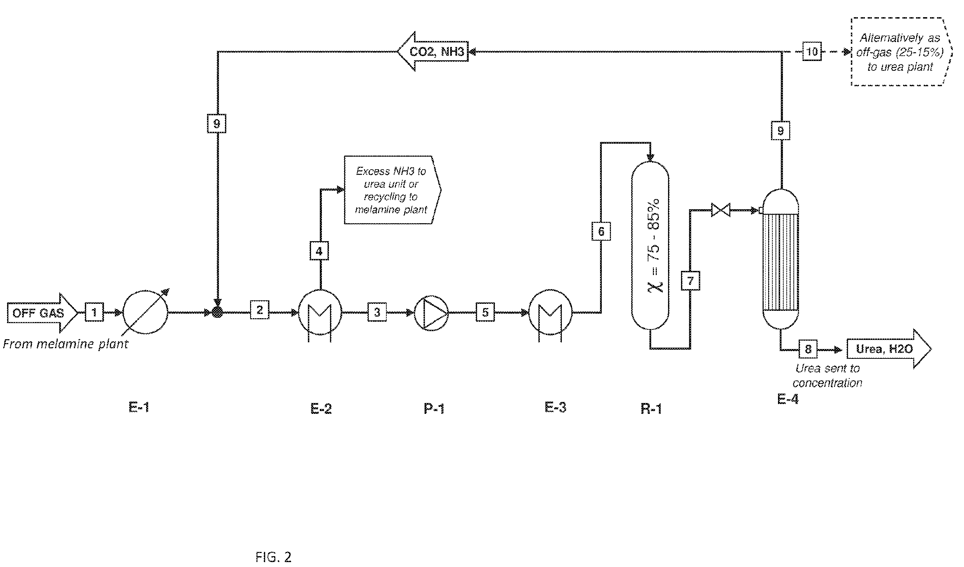

In Figure 2, a simplified diagram of a first embodiment of the process according to the present invention is represented, namely the case in which, in step b) of the process according to the present invention, the off-gases are condensed in liquid phase in the form of a liquid solution of ammonium carbamate and NH₃, the operating pressure of the melamine synthesis process being higher than the triple point pressure of the ammonium carbamate.

In accordance with Figure 2, the anhydrous off-gas stream, at a pressure higher than the triple point pressure of the ammonium carbamate, and lower than 120 bars, coming from the melamine reactor (1), is sent to a gas cooler or off-gas cooling device (E-1). The off-gas stream thus cooled (stream 2) leaves the gas-cooler (E-1) and is sent to a carbamate condenser or surface condenser (E-2) where the off-gases are condensed in liquid phase in the form of a liquid solution of ammonium carbamate and NH₃. The stream (3) of liquid carbamate is sent to a pressurization pump P-1, where it is pressurized at the urea synthesis pressure (145-220 bars), whereas the stream of excess ammonia (4) is recovered in gas phase and recycled in a position suitable for the melamine plant or urea plant. The liquid stream of pressurized ammonium carbamate (5) is then sent to a heating element (heater - E-3) where it is brought to a temperature suitable for the synthesis of urea (180-200°C).

The stream (6) of anhydrous ammonium carbamate thus obtained in liquid phase and under pressure and temperature conditions suitable for the efficient formation of urea (T = 180-200°C, P = 145-220 bars), is thus introduced into a plug-flow reactor (R-1), preferably fed from above (down-flow) with a residence time of a few tens of minutes in order to reach equilibrium.

The product obtained from the urea synthesis reactor (7) is sent to a decomposer (stripper E-4) where the urea is separated from most of the unreacted CO₂ and NH₃ which are recycled (stream 9), after being joined with stream (2), to the condenser E-2, whereas the bottom stream (8) of the decomposer, consisting of urea (about 73% wt) and synthesis water (about 22% wt), with small quantities of NH₃ and CO₂ (<5%) is sent to a normal Urea concentration section, not shown in Figure 2.

Alternatively, the stream (10) of unreacted reagents separated at the head of the decomposer E-4 can be recycled to the Urea plant in the form of an anhydrous gas mixture of NH₃ and CO₂, having a composition similar to that of the starting off-gases, but in significantly reduced quantities (25-15%).

In Figure 3, a simplified diagram of a second embodiment of the process according to the present invention is represented, namely the case in which, in step b) of the process according to the present invention, CO₂ and NH₃ desublimate in solid phase in the form of solid ammonium carbamate:

(2+x) NH₃ (g) + CO₂ (g) → NH₂CONH₄ (l,s) + x NH₃ (g)

the operating pressure of the melamine synthesis process being lower than the triple point pressure of the ammonium carbamate.

In accordance with Figure 3, the anhydrous off-gas stream coming from the melamine reactor (1') is sent to a gas-cooler or off-gas cooling device (E-1). The off-gas stream thus cooled (stream 2') leaves the gas-cooler (E-1) and is sent to a pair of desublimators (E-2-AB), which operate alternately in the desublimation phase (Cooling-Mode ) and pressurization/melting (Heating-Mode), where CO₂ and NH₃ desublimate in solid phase in the form of solid ammonium carbamate and the excess ammonia is recovered in gas phase (4') and recycled in a position suitable for the melamine plant or urea plant. The stream (3') of liquid ammonium carbamate is pressurized at the urea synthesis pressure (> 150 bars) with a discontinuous process by pressurization with CO₂ and NH₃ in the desublimator itself. The stream (3') of pressurized carbamate is therefore a liquid carbamate stream (5') which is sent by means of a pump P-2 to a heating element (heater - E-3) where it is brought to a temperature suitable for the synthesis of urea ranging from 180 to 200°C.

The stream (6') of anhydrous ammonium carbamate thus obtained in liquid phase and under pressure and temperature conditions suitable for the efficient formation of urea (T = 180-200°C, P = 145-220 bars), is thus introduced into a plug-flow reactor (R-1), preferably fed from above (down-flow) with a residence time of a few tens of minutes in order to reach equilibrium.

The product obtained from the urea synthesis reactor (7') is sent to a decomposer (stripper E-4) where the urea is separated from most of the unreacted CO₂ and NH₃ which are recycled (stream 9'), after being joined with stream (2'), to the condenser E-2, whereas the bottom stream (8') of the decomposer, consisting of urea (about 73% wt) and synthesis water (about 22% wt), with small quantities of NH₃ and CO₂ (<5%) is sent to a normal Urea concentration section, not shown in Figure 3.

Alternatively, the stream (10') of unreacted reagents separated at the head of the decomposer E-4 can be recycled to the Urea plant in the form of an anhydrous gas mixture of NH₃ and CO₂, having a composition similar to that of the starting off-gases, but in significantly reduced quantities (25-15%).

## Claims

1. A conversion process of an anhydrous off-gas stream coming from melamine synthesis plants, at a pressure lower than 120 bars, into urea, comprising the following steps:
a) a first isobar cooling step of the off-gas stream;
b) a transformation step of the cooled off-gas stream coming from step a) into ammonium carbamate and NH₃;
c) an isothermal pressurization step of the ammonium carbamate obtained in the previous step;
d) an isobar heating step of the product coming from step c);
e) a conversion step of the ammonium carbamate coming from step d) into urea.

2. The process according to claim 1, wherein step a) is carried out on an anhydrous off-gas stream coming from the melamine synthesis reactor or from the post-reactor possibly present in the melamine plant, or from both.

3. The process according to one or more of the previous claims, wherein step a) also comprises a subsequent purification step of the cooled off-gas stream from the melamine residues contained therein.

4. The process according to one or more of the previous claims, wherein step b) consists of a condensation or a desublimation of the off-gas stream coming from step a).

5. The process according to claim 4, wherein step b) is a condensation of the off-gas stream to give ammonium carbamate and NH₃ in the form of a liquid solution.

6. The process according to claim 4, wherein step b) is a desublimation of the off-gas stream to give ammonium carbamate and NH₃.

7. The process according to one of claims 4 or 5, wherein the excess ammonia produced in step b) is recycled to the melamine synthesis plant or to a urea synthesis plant.

8. The process according to one or more of the previous claims, wherein step c) consists of an isothermal pressurization step wherein the anhydrous, liquid or solid, ammonium carbamate, obtained in the previous step b), is pressurized at a pressure ranging from 145 to 220 bars, in continuous or batchwise.

9. The process according to one or more of the previous claims, wherein step d) consists of an isobar heating step wherein the anhydrous ammonium carbamate, coming from step c), is heated to a reaction temperature T ranging from 180 to 200°C.

10. The process according to one or more of the previous claims, wherein step e) consists of a conversion step of the ammonium carbamate coming from step d) into urea wherein the anhydrous ammonium carbamate is converted into urea with a conversion ranging from 75 to 85%.

11. The process according to one or more of the previous claims, wherein the anhydrous off-gases, coming from melamine synthesis plants, have a pressure lower than 120 bars, preferably lower than 100 bars and even more preferably within the range of 60-100 bars.

12. A plant for the direct conversion of an anhydrous off-gas stream coming from melamine synthesis plants, at a pressure lower than 120 bars, into urea which comprises:
- a cooling device suitable for cooling an off-gas stream coming from the melamine synthesis plant;
- a condenser or at least a pair of desublimators, respectively suitable for condensing or desublimating and pressurizing an off-gas stream coming from the cooling device into ammonium carbamate and ammonia;
- a pump suitable for pressurizing the ammonium carbamate coming from the condenser at a pressure ranging from 145 to 220 bars and sending said ammonium carbamate to a heating element or a pump suitable for sending the ammonium carbamate coming from the desublimator to said heating element;
- a heating element suitable for heating the pressurized ammonium carbamate to a reaction temperature T ranging from 180 to 200°C;
- a reactor suitable for converting the ammonium carbamate into urea.

13. The plant according to claim 12, which comprises the pair of desublimators which send the ammonium carbamate directly to the heating element, without a pump.

## Patentansprüche

1. Umwandlungsverfahren eines aus Melaminsyntheseanlagen kommenden wasserfreien Abgasstroms bei einem Druck von weniger als 120 bar in Harnstoff, umfassend die folgenden Schritte:
a) einen ersten isobaren Kühlschritt des Abgasstroms;
b) einen Umwandlungsschritt des aus Schritt a) kommenden gekühlten Abgasstroms in Ammoniumcarbamat und NH₃;
c) einen isothermen Druckbeaufschlagungsschritt des im vorherigen Schritt erhaltenen Ammoniumcarbamats;
d) einen isobaren Erhitzungsschritt des aus Schritt c) kommenden Produkts;
e) einen Umwandlungsschritt des aus Schritt d) kommenden Ammoniumcarbamats in Harnstoff.

2. Verfahren nach Anspruch 1, wobei Schritt a) an einem wasserfreien Abgasstrom durchgeführt wird, der aus dem Melaminsynthesereaktor oder aus dem gegebenenfalls in der Melaminanlage vorhandenen Nachreaktor oder aus beiden kommt.

3. Verfahren nach einem oder mehreren der vorhergehenden Ansprüche, wobei Schritt a) auch einen anschließenden Reinigungsschritt des abgekühlten Abgasstroms von den darin enthaltenen Melaminrückständen umfasst.

4. Verfahren nach einem oder mehreren der vorhergehenden Ansprüche, wobei Schritt b) aus einer Kondensation oder einer Desublimation des aus Schritt a) kommenden Abgasstroms besteht.

5. Verfahren nach Anspruch 4, wobei Schritt b) eine Kondensation des Abgasstroms zu Ammoniumcarbamat und NH₃ in Form einer flüssigen Lösung ist.

6. Verfahren nach Anspruch 4, wobei Schritt b) eine Desublimation des Abgasstroms zu Ammoniumcarbamat und NH₃ ist.

7. Verfahren nach einem der Ansprüche 4 oder 5, wobei das in Schritt b) produzierte überschüssige Ammoniak in die Melaminsyntheseanlage oder in eine Harnstoffsyntheseanlage recycelt wird.

8. Verfahren nach einem oder mehreren der vorhergehenden Ansprüche, wobei Schritt c) aus einem isothermen Druckbeaufschlagungsschritt besteht, wobei das wasserfreie, flüssige oder feste Ammoniumcarbamat, das im vorhergehenden Schritt b) erhalten wurde, bei einem Druck im Bereich von 145 bis 220 bar kontinuierlich oder diskontinuierlich mit Druck beaufschlagt wird.

9. Verfahren nach einem oder mehreren der vorhergehenden Ansprüche, wobei Schritt d) aus einem isobaren Erhitzungsschritt besteht, wobei das aus Schritt c) kommende wasserfreie Ammoniumcarbamat auf eine Reaktionstemperatur T im Bereich von 180 bis 200 °C erhitzt wird.

10. Verfahren nach einem oder mehreren der vorhergehenden Ansprüche, wobei Schritt e) aus einem Umwandlungsschritt des aus Schritt d) kommenden Ammoniumcarbamats in Harnstoff besteht, wobei das wasserfreie Ammoniumcarbamat in Harnstoff mit einer Umwandlung im Bereich von 75 bis 85 % umgewandelt wird.

11. Verfahren nach einem oder mehreren der vorhergehenden Ansprüche, wobei die wasserfreien Abgase, die aus Melaminsyntheseanlagen kommen, einen Druck von weniger als 120 bar, vorzugsweise weniger als 100 bar und noch bevorzugter im Bereich von 60-100 bar aufweisen.

12. Anlage zur direkten Umwandlung eines aus Melaminsyntheseanlagen kommenden wasserfreien Abgasstroms bei einem Druck von weniger als 120 bar in Harnstoff, umfassend:
- eine Kühlvorrichtung, die zum Kühlen eines aus der Melaminsyntheseanlage kommenden Abgasstroms geeignet ist;
- einen Kondensator oder mindestens ein Paar Desublimatoren, die jeweils geeignet sind, einen aus der Kühlvorrichtung kommenden Abgasstrom in Ammoniumcarbamat und Ammoniak zu kondensieren oder desublimieren und mit Druck zu beaufschlagen;
- eine Pumpe, die geeignet ist, das aus dem Kondensator kommende Ammoniumcarbamat bei einem Druck im Bereich von 145 bis 220 bar mit Druck zu beaufschlagen und das Ammoniumcarbamat einem Heizelement zu senden oder eine Pumpe, die geeignet ist, das Ammoniumcarbamat vom Desublimator zum Heizelement zu senden;
- ein Heizelement, das geeignet ist, das unter mit Druck beaufschlagte Ammoniumcarbamat auf eine Reaktionstemperatur T im Bereich von 180 bis 200 °C zu erhitzen;
- einen Reaktor, der zur Umwandlung des Ammoniumcarbamats in Harnstoff geeignet ist.

13. Anlage nach Anspruch 12, die das Paar Desublimatoren umfasst, die das Ammoniumcarbamat ohne Pumpe direkt zum Heizelement senden.

## Revendications

1. Procédé de conversion en urée d'un flux d'effluents gazeux anhydres issu d'usines de synthèse de mélamine, à une pression inférieure à 120 bars, comprenant les étapes suivantes :
a) une première étape de refroidissement isobare du flux d'effluents gazeux ;
b) une étape de transformation du flux d'effluents gazeux refroidi issu de l'étape a) en carbamate d'ammonium et NH₃ ;
c) une étape de pressurisation isotherme du carbamate d'ammonium obtenu à l'étape précédente ;
d) une étape de chauffage isobare du produit issu de l'étape c) ;
e) une étape de conversion du carbamate d'ammonium issu de l'étape d) en urée.

2. Procédé selon la revendication 1, dans lequel l'étape a) est réalisée sur un flux d'effluents gazeux anhydres issu du réacteur de synthèse de la mélamine ou du post-réacteur éventuellement présent dans l'usine de mélamine, ou des deux.

3. Procédé selon l'une ou plusieurs des revendications précédentes, dans lequel l'étape a) comprend également une étape ultérieure de purification du flux d'effluents gazeux refroidi des résidus de mélamine qu'il contient.

4. Procédé selon l'une ou plusieurs des revendications précédentes, dans lequel l'étape b) consiste en une condensation ou une désublimation du flux d'effluents gazeux issu de l'étape a).

5. Procédé selon la revendication 4, dans lequel l'étape b) est une condensation du flux d'effluents gazeux pour donner du carbamate d'ammonium et du NH₃ sous la forme d'une solution liquide.

6. Procédé selon la revendication 4, dans lequel l'étape b) est une désublimation du flux d'effluents gazeux pour donner du carbamate d'ammonium et du NH₃.

7. Procédé selon une des revendications 4 ou 5, dans lequel l'excédent d'ammoniac produit à l'étape b) est recyclé dans l'usine de synthèse de mélamine ou dans une usine de synthèse d'urée.

8. Procédé selon une ou plusieurs des revendications précédentes, dans lequel l'étape c) consiste en une étape de pressurisation isotherme dans laquelle le carbamate d'ammonium anhydre, liquide ou solide, obtenu à l'étape b) précédente, est pressurisé à une pression allant de 145 à 220 bars, en continu ou par lots.

9. Procédé selon une ou plusieurs des revendications précédentes, dans lequel l'étape d) consiste en une étape de chauffage isobare dans laquelle le carbamate d'ammonium anhydre, issu de l'étape c), est chauffé à une température de réaction T comprise entre 180 et 200°C.

10. Procédé selon une ou plusieurs des revendications précédentes, dans lequel l'étape e) consiste en une étape de conversion du carbamate d'ammonium issu de l'étape d) en urée, dans laquelle le carbamate d'ammonium anhydre est converti en urée avec une conversion comprise entre 75 et 85 %.

11. Procédé selon une ou plusieurs des revendications précédentes, dans lequel les effluents gazeux anhydres, issus des usines de synthèse de mélamine, ont une pression inférieure à 120 bars, de préférence inférieure à 100 bars et encore plus préférentiellement comprise entre 60 et 100 bars.

12. Usine pour la conversion directe en urée d'un flux d'effluents gazeux anhydres issu d'usines de synthèse de mélamine, à une pression inférieure à 120 bars, qui comprend :
- un dispositif de refroidissement pouvant refroidir le flux d'effluents gazeux issu de l'usine de synthèse de mélamine ;
- un condenseur ou au moins une paire de désublimateurs, respectivement adaptés à la condensation ou à la désublimation et à la pressurisation d'un flux d'effluents gazeux issu du dispositif de refroidissement en carbamate d'ammonium et en ammoniac ;
- une pompe pouvant pressuriser le carbamate d'ammonium provenant du condenseur à une pression comprise entre 145 et 220 bars et envoyer ledit carbamate d'ammonium vers un élément chauffant ou une pompe pouvant envoyer le carbamate d'ammonium issu du désublimateur vers ledit élément chauffant ;
- un élément chauffant pouvant chauffer le carbamate d'ammonium sous pression à une température de réaction T comprise entre 180 et 200°C ;
- un réacteur pouvant transformer le carbamate d'ammonium en urée.

13. Usine selon la revendication 12, qui comprend la paire de désublimateurs qui envoie le carbamate d'ammonium directement à l'élément chauffant, sans pompe.
